# FASCICULE DE BREVET EUROPEEN

(11) **EP 4 680 794 B1**
(45) Date de publication et mention de la délivrance du brevet: **28.10.2026**
(21) Numéro de dépôt: 24711533.0
(22) Date de dépôt: 13.03.2024
(51) Int. Cl.: D04H 3/007, A61B 50/20, A61B 50/30, D04H 3/14

(54) **POCHETTE DE STÉRILISATION COMPORTANT UN NON-TISSÉ POREUX**
STERILISATIONSBEUTEL UMFASSEND EINEN PORÖSEN VLIESSTOFF
STERILIZATION POUCH COMPRISING A POROUS NONWOVEN

(30) Priorité: 13.03.2023 FR 2302304
(43) Date de publication de la demande: 21.01.2026
(73) Titulaire: Sterimed SAS, 66110 Amelie-les-Bains-Palalda (FR)
(72) Inventeur: CORNET, Eva, 66110 Amélie-les-Bains-Palalda (FR); AUFFRAY, Clémentine, 66110 Amelie-les-Bains-Palalda (FR); SABARD, Mathieu, 66110 Amelie-les-Bains-Palalda (FR)
(74) Mandataire: Ipsilon
(86) Numéro de dépôt international: PCT/EP2024/056637
(87) Numéro de publication internationale: WO 2024/189066

(56) Documents cités:
- US-A1- 2018 327 948
- US-A1- 2022 195 645
- US-B2- 6 670 035
- PUBCHEM: "Titanium Dioxide | TiO2 | CID 26042 - PubChem", 26 March 2005 (2005-03-26), XP093076410, Retrieved from the Internet <URL:https://pubchem.ncbi.nlm.nih.gov/compound/26042#section=MeSH-Entry-Terms> [retrieved on 20230825]

## Description

### Domaine technique

La présente invention concerne les matériaux non-tissés utilisés dans la confection d'articles de stérilisation, et plus particulièrement mais non exclusivement dans la confection des systèmes de barrière stérile préformés, notamment les pochettes de stérilisation.

### Technique antérieure

Pour stériliser le matériel médical (réutilisable ou à usage unique), il est connu d'utiliser des systèmes de barrière stérile préformés (pré-SBS) répondant à certaines normes, dont notamment les normes ISO11607 (Emballages des dispositifs médicaux stérilisés au stade terminal) et NF EN 868, notamment partie 9, qui décrit les exigences et les méthodes d'essai pour les matériaux non-tissés à base de polyoléfines non enduits. Ces systèmes de barrière stérile préformés peuvent être constitués d'un film polymère, notamment multicouche, classiquement à base de Polypropylène (PP) et de Polyéthylène téréphtalate (PET), et d'un matériau non-tissé poreux et mis en œuvre par thermoscellage. Un côté du pré-SBS n'est pas scellé, afin de laisser une ouverture pour permettre l'introduction de l'instrument à stériliser. Une fois celui-ci inséré, le système de barrière stérile préformé peut être scellé à chaud au niveau de son ouverture, et l'ensemble placé dans une enceinte de stérilisation telle qu'un autoclave ou une chambre de stérilisation, où un agent de stérilisation est injecté. Cet agent peut être de la vapeur d'eau, du formaldéhyde, du peroxyde d'hydrogène (notamment excité par champ électromagnétique - on parle alors de stérilisation par gaz- plasma) ou de l'oxyde d'éthylène. La stérilisation peut aussi se faire par irradiation.

La porosité du non-tissé permet ainsi à l'agent stérilisant de pénétrer dans le système pour stériliser son contenu.

Lorsque l'instrument doit être utilisé, le personnel médical procède à l'ouverture du système par séparation du non-tissé et du film polymère ; lors de cette opération, il est souhaitable qu'aucune fibre du non-tissé ne soit soulevée, afin de garantir l'asepsie du système d'emballage. En effet, un soulèvement de fibres peut entrainer un risque de pollution du champ opératoire.

La force de scellage entre le film polymère et le non-tissé doit donc être suffisante pour garantir l'intégrité du contenu stérile du système et résister aux mises sous pression lors des cycles de stérilisation, mais pas trop élevée pour éviter un risque de déchirement et de libération de fibres à l'ouverture.

La demande GB2449418 décrit l'utilisation de polypropylène (PP) métallocène pour fabriquer un non-tissé poreux utilisable pour la réalisation d'emballages stérilisables. Il n'est pas prévu de charges autres que des composés fluorés, des agents antistatiques, de coloration ou antimicrobiens.

La demande US2018/0327948A1 décrit un article extensible non tissé de type SMMS comportant des couches de type filé-lié«( spunbound) » contenant un polypropylène métallocène mélangé à d'autres polymères tels que du PE, du PC et 0,25% en poids de TiO₂. Un tel matériau ne convient pas à la réalisation de pochettes de stérilisation, car il ne répond pas à la norme NF EN 868-9, en termes de résistance mécanique et d'ouverture aseptique notamment. Cette norme prévoit au point 2.2.1 une résistance en traction sens machine MD (« machine direction ») d'au moins 2,5 kN/m et en sens travers CD (« cross direction ») d'au moins 1,5 kN/m.

La demande US2022/0195645A1 décrit un non-tissé comportant des fibres bi-composant comprenant des polymères pouvant comportant du PP métallocène, avec une charge de l'ordre de plusieurs % en masse de CaCO₃ et de TiO₂.

### Exposé de l'invention

Il existe un besoin pour perfectionner encore les non-tissés poreux utilisés dans la confection d'articles de stérilisation, notamment de systèmes de barrière stérile préformés, afin d'en faciliter l'ouverture par pelage tout en garantissant une force de scellage suffisante, une bonne qualité de l'empreinte laissée sur le non tissé et une absence de déchirure pour garantir l'asepsie.

### Résumé de l'invention

L'invention vise à répondre à ce besoin et elle y parvient en proposant une pochette de stérilisation selon la revendication 1, comportant un non-tissé poreux destiné à la réalisation d'articles de stérilisation, notamment de systèmes de barrière stérile préformés, comportant des fibres formées au moins partiellement de polypropylène métallocène, et entre 0,1 et 1,4% de dioxyde de titane en masse par rapport au poids total du non-tissé, mieux entre 0,1 et 0,8%.

Grâce à l'invention, les forces de scellage peuvent être ajustées. Ainsi la pelabilité et le ressenti de l'utilisateur à l'ouverture du système de barrière stérile peuvent être améliorés. Les forces à l'ouverture sont constantes et modérées ce qui garantit une facilité d'ouverture, une absence de déchirure du non tissé ainsi qu'un maintien de l'asepsie. La qualité de l'empreinte formée sur le non-tissé à l'ouverture du système de barrière stérile est également améliorée (l'empreinte peut être plus homogène, plus blanche, sans zones transparentes (c'est-à-dire sans transparentisation), et ne présente pas de fibres soulevées).

De manière surprenante, la teneur revendiquée permet d'adapter et d'améliorer les performances du scellage, tout en maintenant de bonnes performances mécaniques et en préservant l'asepsie.

En effet, une teneur supérieure à 1,4 % en masse de dioxyde de titane conduit notamment à une diminution significative de la résistance à la déchirure dans le sens marche, ainsi qu'à une diminution de la résistance à la traction dans les sens marche et transverse.

Une teneur inférieure à 0,1 % conduit à une empreinte transparente sur le non-tissé poreux, notamment à des températures de scellage comprises entre 150°C et 160°C. De plus, les forces de scellage ne sont pas ajustées, ce qui peut provoquer des déchirures du non tissé, ou des soulèvements de fibres à l'ouverture du système de barrière stérile.

Le non-tissé selon l'invention répond aux normes ISO 11607 et ISO 10993. Il est également conforme aux normes DIN 58953-6 (cette dernière décrit les essais de propriétés de barrière microbienne pour les dispositifs médicaux devant être stérilisés), ASTM F2101 (mesure de l'efficacité de la filtration bactérienne BFE) et ASTM F1608 (mesure la capacité d'un matériau poreux à empêcher la pénétration des bactéries). Selon cette dernière méthode, un échantillon témoin totalement imperméable (pénétration bactérienne = 0) est exposé à un million cfu (unités formant colonie). Le nombre 10⁶ possède une valeur de log de 6. Si un échantillon soumis au même test que l'échantillon témoin permet la pénétration de 10¹ cfu (log 10 = 1), sa valeur LRV (Log Reduction Value) est 5 (6 - 1 = 5). Ainsi, plus la valeur LRV est élevée, plus le matériau poreux sera résistant aux bactéries et aux micro-organismes.

Lorsque le non-tissé sert à la réalisation d'une pochette de stérilisation, le matériau non tissé doit répondre aux exigences de la norme NF EN 868-9. En particulier, la pochette doit présenter une ouverture aseptique et le non-tissé présenter une résistance à la traction sens machine MD (mesurée selon EN ISO 1924-2) d'au moins 2,5 kN/m et sens travers CD (mesurée selon EN ISO 1924-2) d'au moins 1,5 kN/m selon cette norme (point 2.2.1) pour les matériaux à base de polypropylène. Selon une ou plusieurs caractéristiques avantageuses de l'invention, considérées isolément ou en combinaison :
- le non-tissé comporte entre 0,1 et 1% en masse de dioxyde de titane, notamment plus de 0,25 % en masse de dioxyde de titane, mieux entre 0,4% et 1% en masse de dioxyde de titane, encore mieux entre 0,4 et 0,6% en masse de dioxyde de titane,
- le non-tissé est du type filé-lié, selon l'invention, le non-tissé est dépourvu d'une autre charge minérale que le dioxyde de titane en une quantité supérieure ou égale à 0,1% en masse, notamment strictement inférieure à 0,1% en masse, voire ne contient que du dioxyde de titane en tant que charge minérale, l'absence de carbonate de calcium comme charge étant avantageuse en ce qu'elle évite la création de faux positifs en biocompatibilité ;
- le dioxyde de titane est de taille moyenne en nombre comprise entre 100 et 1000 nm,
- les fibres sont formées entièrement, en ce qui concerne le ou les matériau(x) polymère(s) qui les constitue(nt), de polypropylène métallocène.

L'invention a encore pour objet, selon un autre de ses aspects, un procédé de fermeture d'une pochette selon l'invention, dans lequel on scelle à chaud le non-tissé sur un support thermoplastique.

Le scellage s'effectue de préférence à une température comprise entre 135°C et 165°C, mieux entre 150°C et 160°C.

### Brève description des dessins

L'invention pourra être mieux comprise à la lecture de la description détaillée qui va suivre, d'exemples non limitatifs de mise en œuvre de celle-ci, et à l'examen du dessin annexé, sur lequel :
- [Fig 1] représente en vue de face, côté non tissé, un exemple de pochette de stérilisation selon l'invention,
- [Fig 2] est une coupe transversale partielle et schématique de la pochette,
- [Fig 3] est un tableau illustrant les caractéristiques mécaniques des non-tissés obtenus pour différentes teneurs de dioxyde de titane,
- [Fig 4] est un tableau illustrant les forces de scellage mesurées et autres constatations, pour différentes températures de scellage et différentes teneurs en dioxyde de titane.

### Description détaillée

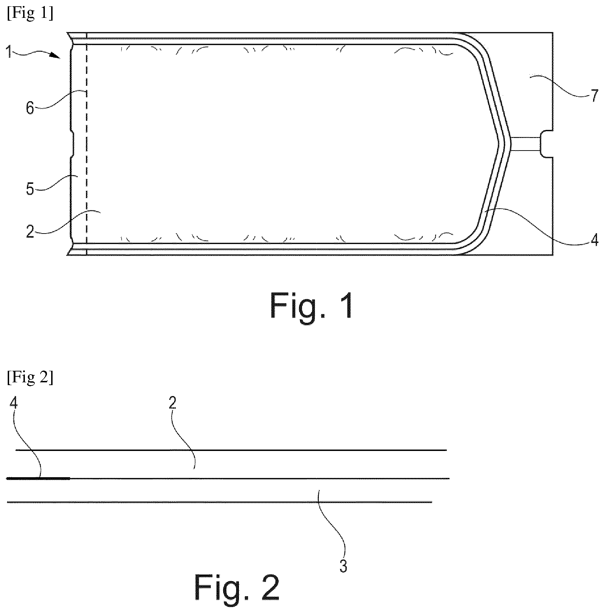

On a représenté aux figures 1 et 2 un exemple de pochette de stérilisation 1 conforme à l'invention, comportant un non tissé poreux 2 thermoscellé sur un film de support 3 en matériau thermoplastique non poreux, à base de polypropylène PP et de PET.

Sur la figure 1, on voit la pochette scellée sur une ligne de soudure 4 s'étendant sur trois côtés seulement, ménageant ainsi une ouverture 5 d'introduction d'un instrument à stériliser. Le non tissé est thermoscellé sur le support selon une ligne de fermeture 6 après introduction de l'instrument, puis la pochette est mise à stériliser.

La pochette 1 présente du côté opposé à l'ouverture 5 une zone 7 où le non tissé recouvre le support sans y être soudé, de façon à permettre leur préhension par les mains de l'utilisateur afin de les écarter lors de l'ouverture par pelage de la pochette.

La stérilisation de ce système de barrière stérile peut s'effectuer selon différentes méthodes :
- par chaleur humide : l'agent stérilisant est la vapeur d'eau (température : 121°C - 134°C pendant de 18 - 30 min, sous pression).
- par l'oxyde d'éthylène : température comprise entre 30 et 60°C, avec une humidié relative supérieure de 30% pendant 16-18 h avec une concentration d'oxyde d'éthylène de 200- 800 mg/L). L'oxyde d'éthylène est un agent toxique, ainsi une période de désorption est à respecter après stérilisation.
- par rayons ionisants (rayons gamma ou stérilisation beta ou faisceau d'électrons).
- par le formaldéhyde : le formol peut se présenter sous forme de gaz.
- par gaz-plasma : utilisation sous forme de gaz de peroxyde d'hydrogène (H₂O₂) diffusé dans l'enceinte et transformé en plasma par un champ électromagnétique. Sont générés des ions, des électrons libres, et de nombreux radicaux libres à très forte activité bactéricide. Cette méthode permet une stérilisation à basse température, et ne nécessite pas de désorption.

### PP métallocène

Au sens de l'invention, le terme « polypropylène » couvre un homopolymère de propylène, les co-polymères de propylène obtenus par copolymérisation du propylène avec au moins un co-monomère de type alpha-oléfine et les dérivés d'homopolymère de propylène ou de copolymères de propylène fonctionnalisés, notamment par greffage d'une fonction réactive suite à une interaction avec l'anhydride maléique ou l'acide acrylique, comme par exemple un polypropylène maléaté. Un copolymère peut être à bloc ou statistique.

Le polypropylène (quelle que soit sa forme) peut être isotactique ou syndiotactique.

Par « PP métallocène » il faut comprendre au sens de l'invention un polypropylène obtenu par catalyse métallocène.

Le polypropylène est réalisé via une catalyse métallocène plutôt que par le procédé Ziegler-Natta (l'utilisation de ce type de catalyse pour la production de polyoléfines est bien connue de l'homme du métier comme décrit dans les brevets US5571619, US5322728 et US5472775).

Le non-tissé est de préférence réalisé à partir de polypropylène (PP) métallocène conformément à l'enseignement du brevet GB2449418.

Le PP métallocène a une distribution de poids moléculaire (MWD) généralement assez étroite, de préférence avec une valeur de MWD de moins de 3, notamment de moins de 2,5, et permet la production de fibres ayant un diamètre moyen inférieur à 25 microns, et notamment dans la plage 10-20 microns.

Le non tissé obtenu est formé par une technique dite de filé-lié (« spun bond »), de façon connue en soi. La formation des fibres de polypropylène métallocène peut se faire comme décrit dans la demande GB2449418 (ce procédé est également décrit dans les documents US3821062, US3563838, US 20210301425 et US2019145032). Le dioxyde de titane (TiO₂) est intégré durant la production des fibres de polypropylène, par mélange avec le PP métallocène, notamment par mélange à sec. Le dioxyde de titane se présente de préférence sous forme de mélange maître (« masterbatch »).

Le PP utilisé peut notamment être le Metocene HM562S d'indice de fluidité à chaud égal à 30g/10' mesuré à 230°C, sous un poids de 2.16kg (selon la norme ASTM D1238).

Selon l'invention, le non tissé poreux peut faire l'objet d'une étape de calandrage. Lors de cette étape de calandrage, plusieurs paramètres peuvent varier, comme la température (typiquement autour de 130°C et 160°C), la vitesse (typiquement supérieure à 10m/min) et la pression (typiquement supérieure à 30 bars).

La teneur massique en dioxyde de titane du non-tissé est comprise entre 0.1 et 1.4%, étant de préférence de l'ordre de 0,5%.

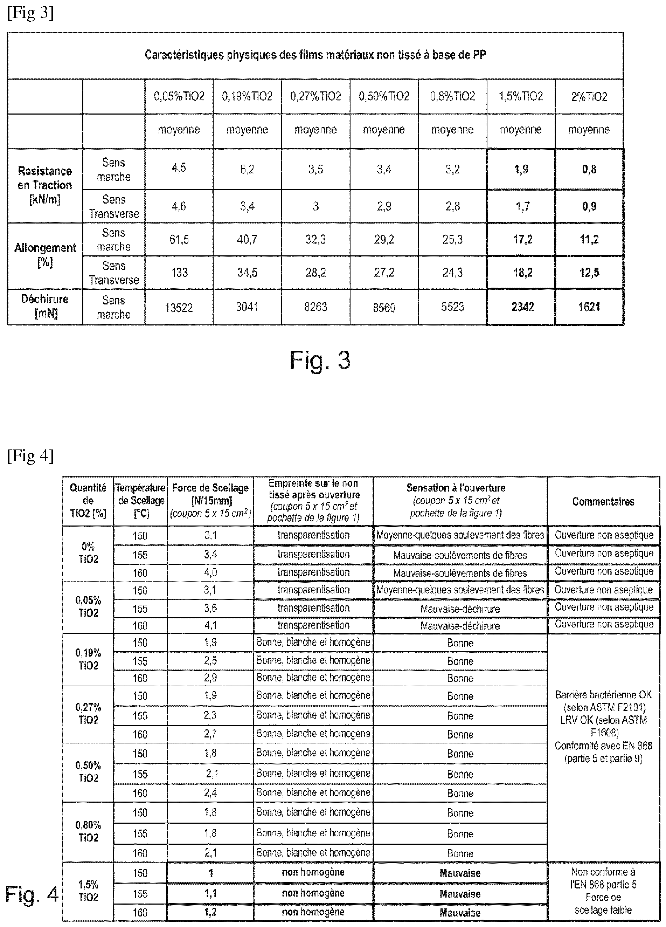

Une telle teneur permet de bénéficier de propriétés avantageuses pour la fabrication de la pochette, comme cela va être détaillé en référence aux tableaux des figures 3 et 4.

Les fibres du non-tissé peuvent avoir un diamètre moyen allant de 10 à 25 microns.

Le dioxyde de titane peut avoir une taille moyenne en nombre allant de 100 nm à 1 µm.

### Essais comparatifs

Différents échantillons à base de non tissé poreux composé de PP métallocène (Metocene HM562S) et de TiO₂ (diamètre moyen égal à 300 nanomètre), et d'un film polymère laminé PET 12µ /PP 40µ ont été réalisés.

Le non-tissé a un grammage de 93 g/m² en moyenne (mesuré selon la norme ISO 536). Des bandes de 5 cm x 15 cm ont été préparées pour chaque échantillon. La face PP du film PET/PP a été thermoscellée sur le non tissé poreux.

Des couples film (PET/PP) - non tissé poreux ont été thermoscellés avec une thermoscelleuse de laboratoire, le scellage s'effectuant avec la mâchoire chaude contre la face PET. Différentes températures de soudage sont testées : 150, 155 et 160°C, à une pression constante de 5 bars, durant un temps de 0,3s.

La mesure des forces de scellage est effectuée sur 10 échantillons, en étant réalisée sur un dynamomètre selon la norme ASTM F88/F88M-2021 (technique B).

Les essais de traction sont réalisés sur dynamomètre selon la norme ASTM D882-18, sur 10 échantillons.

Les essais de déchirure sont réalisés sur 10 échantillons, sur dynamomètre selon la norme ASTM D1938-19.

Ces couples film (PET/PP) - non tissé poreux scellés sur thermoscelleuse de laboratoire ont également permis d'étudier la qualité de l'empreinte à l'ouverture.

Des pochettes telles que celles présentées sur la figure 1 ont également été réalisées sur une machine FFS («Form-Fill-Seal »). 3 températures de scellage ont été utilisées : 150, 155 et 160°C et la cadence fixée à 15 pochettes par minute. Ces pochettes ont permis d'évaluer les sensations à l'ouverture ainsi que la qualité de l'empreinte.

Sur le tableau de la figure 3, on voit que la résistance à la déchirure est fortement dégradée pour les essais à des teneurs de 1,5% ou plus, et qu'elle passe par un maximum relatif à 0,5%.

Sur le tableau de la figure 4, on a comparé les forces de scellage pour différentes températures de scellage et différentes teneurs massiques en dioxyde de titane.

Les résultats sont aussi appréciés sous la forme de commentaires dans les 2 dernières colonnes du tableau de la figure 4

On peut constater pour une teneur inférieure à 0,1 % un manque de blancheur de l'empreinte, et la présence d'un aspect transparent, pour les températures de scellage égales à 150, 155 et 160°C.

On observe une absence de «transparentisation » après pelage, une bonne sensation à l'ouverture, tout en préservant l'asepsie, pour des taux de dioxyde de titane de 0,19, 0,27, 0,5 et 0,8 %.

Les sensations à l'ouverture sont mauvaises à une teneur de 1,5%.

Les meilleurs résultats sont obtenus pour des teneurs de 0.27% et 0.5% en poids de dioxyde de titane, à 155°C et 160°C. En effet, on observe une très bonne sensation à l'ouverture ainsi qu'une empreinte homogène et blanche, en préservant l'asepsie, ainsi que de très bonnes performances mécaniques (résistance à la déchirure notamment).

Bien entendu, l'invention n'est pas limitée aux exemples qui viennent d'être décrits.

On peut mélanger le PP métallocène à au moins un autre thermoplastique, par exemple du polyéthylène (basse densité, ou basse densité linéaire), greffé, ou non greffé, ou du polybutène, entre autres possibilités... La proportion du ou des autres polymères du mélange est choisie pour maintenir les propriétés recherchées.

## Revendications

1. Pochette de stérilisation, comportant un non-tissé poreux comportant des fibres formées au moins partiellement de polypropylène métallocène, et entre 0,1 et 1,4% de dioxyde de titane en masse par rapport au poids total du non-tissé, le non-tissé étant dépourvu d'une autre charge minérale que le dioxyde de titane en une quantité supérieure ou égale à 0,1% en masse.

2. Pochette selon la revendication 1, le non tissé comportant entre 0,1 et 1% en masse de dioxyde de titane.

3. Pochette selon la revendication 1 ou 2, le non tissé comportant entre 0,4% et 1% en masse de dioxyde de titane.

4. Pochette selon l'une quelconque des revendications 1 à 3, le non tissé comportant entre 0,4 et 0,6% en masse de dioxyde de titane.

5. Pochette selon l'une quelconque des revendications précédentes, le non tissé étant du type filé- lié.

6. Pochette selon l'une quelconque des revendications précédentes, le dioxyde de titane étant de taille moyenne en nombre comprise entre 100 et 1000 nm.

7. Pochette selon l'une quelconque des revendications précédentes, les fibres étant formées entièrement, en ce qui concerne le ou les matériau(x) polymère(s) qui les constitue(nt), de polypropylène métallocène.

8. Pochette selon l'une quelconque des revendications précédentes, le non-tissé présentant une résistance à la traction sens machine MD (mesurée selon EN ISO 1924-2) d'au moins 2,5 kN/m et sens travers CD (mesurée selon EN ISO 1924-2) d'au moins 1,5 kN/m selon cette norme (point 2.2.1).

9. Procédé de fermeture d'une pochette selon l'une quelconque des revendications précédentes, dans lequel on scelle à chaud le non-tissé sur un support thermoplastique.

10. Procédé selon la revendication 9, le scellage s'effectuant à une température comprise entre 135°C et 165°C, mieux entre 150°C et 160°C.

## Patentansprüche

1. Sterilisationsbeutel, umfassend einen porösen Vliesstoff, der Fasern, die zumindest teilweise aus Metallocen-Polypropylen gebildet sind, und zwischen 0,1 und 1,4 Gew.-% Titandioxid umfasst, bezogen auf das Gesamtgewicht des Vliesstoffs, wobei der Vliesstoff frei von einem von dem Titandioxid verschiedenen mineralischen Füllstoff in einer Menge größer oder gleich 0,1 Gew.-% ist.

2. Beutel nach Anspruch 1, wobei der Vliesstoff zwischen 0,1 und 1 Gew.-% Titandioxid umfasst.

3. Beutel nach Anspruch 1 oder 2, wobei der Vliesstoff zwischen 0,4 Gew.-% und 1 Gew.-% Titandioxid umfasst.

4. Beutel nach einem der Ansprüche 1 bis 3, wobei der Vliesstoff zwischen 0,4 und 0,6 Gew.-% Titandioxid umfasst.

5. Beutel nach einem der vorhergehenden Ansprüche, wobei der Vliesstoff vom Spinnvlies-Typ ist.

6. Beutel nach einem der vorhergehenden Ansprüche, wobei das Titandioxid eine zahlenmittlere Größe von 100 bis 1000 nm aufweist.

7. Beutel nach einem der vorhergehenden Ansprüche, wobei die Fasern in Bezug auf das Polymermaterial oder die Polymermaterialien, woraus sie hergestellt sind, vollständig aus Metallocen-Polypropylen gebildet sind.

8. Beutel nach einem der vorhergehenden Ansprüche, wobei der Vliesstoff eine Zugfestigkeit in Maschinenrichtung MD (gemessen nach EN ISO 1924-2) von mindestens 2,5 kN/m und eine Zugfestigkeit in Querrichtung CD (gemessen nach EN ISO 1924-2) von mindestens 1,5 kN/m gemäß dieser Norm (Abschnitt 2.2.1) aufweist.

9. Verfahren zum Verschließen eines Beutels nach einem der vorhergehenden Ansprüche, wobei der Vliesstoff mit einem thermoplastischen Träger heißversiegelt wird.

10. Verfahren nach Anspruch 9, wobei das Versiegeln bei einer Temperatur zwischen 135 °C und 165 °C, bevorzugter zwischen 150 °C und 160 °C durchgeführt wird.

## Claims

1. Sterilization pouch comprising a porous nonwoven fabric comprising fibres formed at least partially from metallocene polypropylene, and between 0.1% and 1.4% of titanium dioxide by mass relative to the total weight of the nonwoven fabric, the nonwoven fabric being free of any mineral filler other than titanium dioxide in an amount greater than or equal to 0.1% by mass.

2. Pouch according to Claim 1, the nonwoven fabric comprising between 0.1% and 1% by mass of titanium dioxide.

3. Pouch according to Claim 1 or 2, the nonwoven fabric comprising between 0.4% and 1% by mass of titanium dioxide.

4. Pouch according to any one of Claims 1 to 3, the nonwoven fabric comprising between 0.4% and 0.6% by mass of titanium dioxide.

5. Pouch according to any one of the preceding claims, the nonwoven fabric being of the spunbonded type.

6. Pouch according to any one of the preceding claims, the titanium dioxide having a number-average size of between 100 and 1000 nm.

7. Pouch according to any one of the preceding claims, the fibres being formed entirely, with regard to the polymer material(s) constituting them, from metallocene polypropylene.

8. Pouch according to any one of the preceding claims, the nonwoven fabric having a tensile strength in the machine direction MD (measured according to EN ISO 1924-2) of at least 2.5 kN/m and in the cross direction CD (measured according to EN ISO 1924-2) of at least 1.5 kN/m according to this standard (section 2.2.1).

9. Process for closing a pouch according to any one of the preceding claims, wherein the nonwoven fabric is heat-sealed to a thermoplastic support.

10. Process according to Claim 9, the sealing being performed at a temperature of between 135°C and 165°C, better still between 150°C and 160°C.
